# EUROPEAN PATENT APPLICATION

(11) **EP 0 858 787 A1**
(43) Date of publication of application: **19.08.1998**
(21) Application number: 97200405.5
(22) Date of filing: 13.02.1997
(51) Int. Cl.: A61F 5/44, A61F 13/58

(54) **Adult incontinence brief with sag-eliminator**

(71) Applicant: KONINKLIJKE UTERMÖHLEN N.V., 3526 KS Utrecht (NL)
(72) Inventor: Van den Bogaert, Philip, 2800 Mechelen (BE)
(74) Representative: Donné, Eddy

(57) **Abstract**

A holder for an absorbent pad comprising an impervious backsheet, said backsheet having a first side and a second side,
- two tape tabs (22), one end of each tape tab being affixed to said backsheet;
- adhesive portions (24) on the other end of each tape tab;
- said tape tab (22) between said adhesive portion (24) and said backsheet, being capable of receiving and holding hooks;
- a hook portion (26) of a hook-and-loop fastener affixed to the outside of said backsheet.

## Description

### BACKGROUND OF THE INVENTION

The present invention relates to disposable sanitary absorbent products such as baby diapers, but more particularly to an absorbent brief for adults suffering from incontinence. In this respect, except for the fact that adult briefs are substantially larger than baby diapers, the general function and use of the invention is the same for diapers. However, the present invention is significantly more effective for large adult incontinence briefs.

The disposable baby diaper has been known to the public for at least 30 years, and over that period of time, significant changes have been made to the construction, materials, and the design to improve the product so that substantially larger volumes and weights of human excrement can be contained.

The early disposable diapers were fastened and held to the body of the wearer with safety pins, but in the early 1970's, several adhesive manufacturers such as Avery, Fasson and 3-M developed tape tabs which would fasten securely to the plastic backsheet of the diaper.

### SUMMARY OF THE INVENTION

In response to the needs and disadvantages of the prior art structures, and to overcome any tendency of the garment to sag as more excrement is discharged into it, the present invention improves not only the fit and comfort of the product, but also provides an economic construction in a manner not heretofore shown or described.

Therefore, one aspect of the present invention is to provide an adult incontinence brief which is easy for the wearer to fit around the body and which will be comfortable and stay in position despite repeated insults of urine and increasing volumes and weight to be carried.

Another aspect of the present invention is utilization of the hook-and-loop principle in a combination not heretofore described, wherein the loop portion of a hook-and-loop system is the elastic waistband itself.

An object of the present invention, therefore, is to provide a system which combines the adhesive and plastic landing strip of prior art with the concept of a hook and loop fastener, previously well-known, but not shown in a combination of the present invention, all to reduce or substantially eliminate any sagging of the diaper around the waist of the wearer.

Another object of the present invention is to provide a sanitary disposable absorbent product which can be modified in sizes to be applicable to garments for both children and adults, and which effectively combines an adhesive/landing strip, and the hook-and-loop principle of prior art, to effectively eliminate the sagging of the diaper as it is filled with urine.

Another object is to provide an incontinent pad for adults which can be easily fitted to the body by the wearer, thus eliminating the need for a care giver.

A further object is to reduce the cost of an effective adult incontinent pad.

With the above and other objects in view, further information and a better understanding of the present invention may be achieved by referring to the following detailed description:

With the above and other objects in view, further information and a better understanding of the present invention may be achieved by referring to the following detailed description.

For the purpose of illustrating the invention, there is shown in the accompanying drawings a form thereof which is at present preferred, although it is to be understood that the various instrumentalities of which the invention consists can be variously arranged and organized, and that the invention is not limited to the precise arrangement and organization so the instrumentalities as herein shown and described.

### BRIEF DESCRIPTION OF THE DRAWINGS

In the drawings, wherein like reference characters indicate like parts:

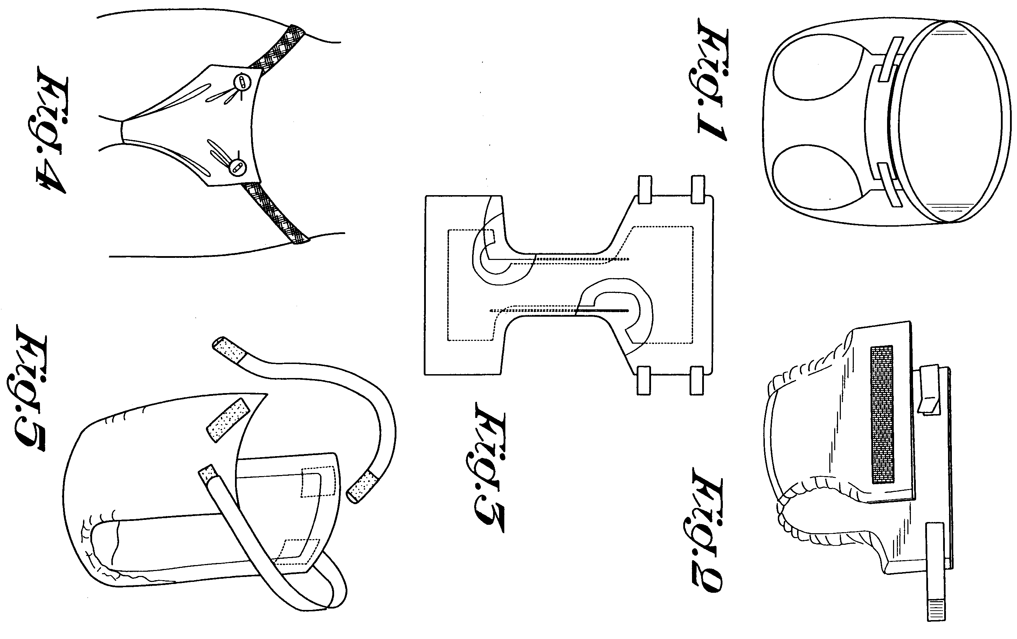

Figure 1 illustrates a perspective view of a fastening system developed by Molnlycke in Sweden, Peaudouce in France, and Unicharm in Japan. It is a frontal strip which can be either embossed or smooth, and onto which the adhesive portion of the tape tab is placed when fitting the diaper around the patient. Because of the size of the "landing strip", a great variety of sizes of patients can be accommodated.

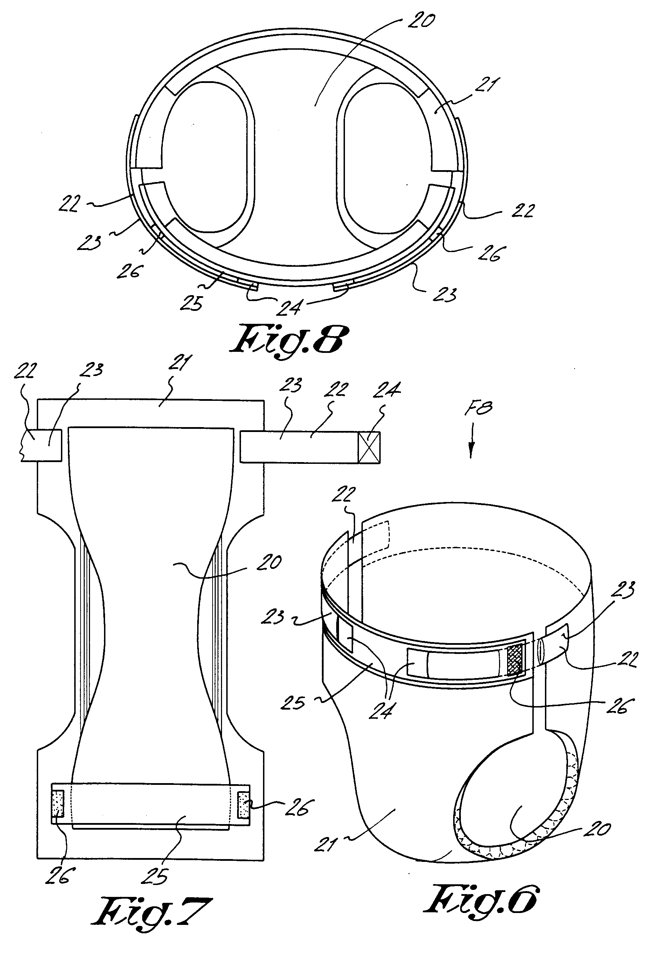

In Figure 2 there is shown a modified form of this arrangement wherein a hook-and-loop combination is used to do the fastening. The hook elements are placed on the tape tab, and the loop elements become the "landing strip". This combination and arrangement is very effective but is quite expensive.

In each of the foregoing fastening arrangements, the waistband portion of the incontinent pad completely encircles the waist of the patient.

The adult brief shown in Figure 3 has been found very effective, because the use of multiple tape tabs and precisely-placed elastic leg bands provide a more effective fecal and urine containment device for adults.

Such devices are expensive, and in order to provide a less expensive device, but one which is still comfortable and cosmetic, the devices shown in figures 4 and 5 have been developed to provide the absorbency where it is needed, and leaving side portions around the hips and legs uncovered.

However, adult devices are designed to fit bigger people and contain a greater volume in weight and body excrement, and this causes the product to sag or droop from the hips of the patient, creating a greater chance for leakage.

Absorbent articles have a tendency to sag or gap away from and to slide or slip down on the body of the wearer. This sagging, gapping and slipping is caused by the relative motions of the wearer, and by the downward forces generated when the absorbent article is loaded with body excrements. This sagging, gapping and slipping can lead to premature leakage and poor fit of the absorbent article about the wearer.

The principle object is to eliminate the sag of the diaper around the waistband.

The arrangement which is shown in figures 6, 7 and 8 utilizes characteristics of fastening systems of previous inventions, but in a manner and direction totally the opposite of that which is taught by the prior art. The cloth-covered elastic waistband of the present invention not only provides the tension necessary to keep the product firmly placed around the body of the wearer, but also becomes the loop element of a hook-and-loop arrangement.

For the purpose of illustrating the invention, there is shown in the accompanying drawings a form thereof which is at present preferred, although it is to be understood that the various instrumentalities of which the invention consists can be variously arranged and organized, and that the invention is not limited to the precise arrangement and organizations of the instrumentalities as herein shown and described.

### BRIEF DESCRIPTION OF THE DRAWINGS

In the drawings wherein like reference characters indicate like parts:
Figure 1 is a prior art incontinence garment utilizing the fastening system taught by the Widlund U.S. Patent 5,370,639.
Figure 2 is a prior art garment using a hook-and-loop fastener in place of the adhesive tape tab and plastic landing strip, as shown in Nestegard U.S. Patent 4,894,068.
Figure 3 is a top plan view of the prior art adult incontinence pad shown in the Strickland U.S. Patent 4,253,461.
Figures 4 and 5 show an adult incontinence pad based on a "loin cloth", one utilizing a button and buttonhole fastening arrangement, and the other a hook-and-loop fastening arrangement.
Figure 6 is a perspective view of the garment of the present invention showing the arrangement which prevents sagging of the diaper around the waist-portion of the wearer.
Figure 7 is a pan view of the incontinence device of the present invention stretched out flat to show the relationship of the adhesive system, as well as the hook-and-loop system.
Figure 8 is a top view taken generally along line 8-8 of Figure 7.

The general construction of the absorbent portion 20 of the pad 21 is similar to the pads of the prior art, with a clear distinction that the elastic waistband members 22 are covered with cloth 23, or covered with a material simulating cloth to provide the loop member of a hook-and-loop fastener.

The end 24 is a patch or piece of adhesive material which is constructed in a well-known manner as is taught in the prior art, so that the adhesive patch 24 can stick to the diaper in the area 25.

Many devices of the prior art have provided an embossed or smooth "landing strip" to receive the adhesive patch 24. In many other devices, as is preferred in the case of the present invention, a reinforcing member (not shown) may be placed on the inner-side of the outer cover as a "reinforcement", and in this case the adhesive patch 24 can be placed directly against the outside of the outer cover.

Furthermore, if a careful relationship between the adhesive material and the plastic of the outer cover is developed so as to provide "strong in shear and weak in peel", then the adhesive patch may be placed anywhere on the outer cover.

In the present invention, it is preferred that a reinforcing member be placed on the inside of the outer cover in a "landing strip" area designated by 25 on Figure 7.

The unique arrangement of the landing strip is its combination of the piece of adhesive 24 which can be secured to the outer cover, as well as the hook portions 26 of a hook-and-loop fastener. This enables the adhesive tape tab arrangements and the hook-and-loop tape tab arrangement to work, not only in combination with each other, but to supplement and provide additional security. More importantly then prevent the waistband portions of the pad to sag down away from the body and create potential leakage areas around the leg of the wearer.

When placing the garment of the present invention on the body, the wearer stretches the elastic bands 22 tightly around the sides (from back to front), and then securing the adhesive portion 24 onto the outside of the outer cover.

Thereafter the cloth-like cover on the elastic straps are pressed tightly against the hook-like portions 26. Inasmuch as the hook-like portions 26 are near the outer edges of the waistband portion at the front of the diaper, those outer edges are prevented from sagging as the pad is loaded with excrement, because they remain tightly "hooked" to the elastic bands.

Thus one can see that the adhesive securement is the primary factor in holding the pad on the body, whereas the hook-and-loop element is a secondary factor but, more importantly, prevents the diaper from sagging on the body of the wearer.

The elastic bands are in tension when the bands are applied to the hooks 26 and remain in tension even under load.

It is further to be understood that the present invention may be embodied in other specific forms without departing from the spirit or special attributes; and it is, therefore, desired that the present embodiments be considered in all respects as illustrative and, therefore, not restrictive, reference being made to the appended claims rather than to the foregoing description to indicate the scope of the invention.

## Claims

1. A fastening system for disposable absorbent sanitary articles comprising a back sheet, a front sheet, and an absorbent core between said back sheet and said front sheet, said article comprising -
- two tape tabs,
- a hook portion of hook-and-loop fastener adjacent each end of the frontal tape,
- each tape tab comprising a manufacturer's end fastened to one end of the back sheet and an adhesive portion at the opposite end thereof,
- said tape tab having a hook-receiving surface.

2. A holder for an absorbent pad comprising an impervious backsheet, said backsheet having a first side and a second side,
- two tape tabs, one end of each tape tab being affixed to said backsheet,
- adhesive portions on the other end of each cape tab,
- said tape tab between said adhesive portion and said backsheet, being capable of receiving and holding hooks,
- a hook portion of a hook-and-loop fastener affixed to the outside of said backsheet.

3. The holder of Claim 2 wherein said holder comprises a topsheet, said backsheet and an absorbent pad between said backsheet and said topsheet.

4. The holder of Claim 3 wherein said adhesive is formulated so that said tape tab is strong in shear and weak in peel when said adhesive is in contact with said backsheet and tension is applied to said tape tab.

5. The holder of Claim 4 wherein the hook portion of said fastener is adapted to engage the hook receiving portion of said hook-and-loop fastener when said adhesive portion is affixed to said backsheet.

6. The holder of Claim 5 wherein said tape tab is in tension only when said adhesive is affixed to said backsheet and said hook portion is affixed to said tape tab between said adhesive and said backsheet.
